Europäisches Patentamt

European Patent Office

Office européen des brevets

(19)

(11) EP 0 686 264 B1

(12) FASCICULE DE BREVET EUROPEEN

(45) Date de publication et mention
de la délivrance du brevet:
**28.05.1997 Bulletin 1997/22**

(51) Int Cl.$^6$: **G01N 33/569**, C12Q 1/04,
C12Q 1/56, C12Q 1/37,
G01N 33/546

(21) Numéro de dépôt: 93905419.3

(22) Date de dépôt: 24.02.1993

(86) Numéro de dépôt international:
**PCT/FR93/00180**

**WO 94/19696 (01.09.1994 Gazette 1994/20)**

(54) **PROCEDE POUR METTRE EN EVIDENCE L'AFFINITE DU FIBRINOGENE ET DE SES DERIVES AUX FORMES FILAMENTAIRES DE LEVURES, UTILISATION DANS LE DOMAINE DE LA DETERMINATION DES LEVURES PATHOGENES NOTAMMENT EN HEMOCULTURE**

VERFAHREN ZUR BESTIMMUNG DER AFFINITÄT VON FIBRINOGEN UND SEINER DERIVATE GEGENÜBER FILAMENTÄREN FORMEN VON HEFEN, UND VERWENDUNG AUF DEM GEBIET DER BESTIMMUNG VON PATHOGENEN HEFEN INSBESONDERE IN BLUTKULTUR

METHOD FOR DETERMINING THE AFFINITY OF FIBRINOGEN AND DERIVATIVES THEREOF FOR FILAMENTARY FORMS OF YEAST, AND USE THEREOF FOR DETERMINING PATHOGENIC YEAST, PARTICULARLY IN BLOOD CULTURE

(84) Etats contractants désignés:
**DE ES IT**

(43) Date de publication de la demande:
**13.12.1995 Bulletin 1995/50**

(73) Titulaire: **DIFFUSION BACTERIOLOGIE DU VAR**
**F-83110 Sanary-sur-Mer (FR)**

(72) Inventeurs:
- **CONTANT-PUSSARD, Geneviève, La Corderie**
  **F-92400 Courbevoie (FR)**
- **DEBRUYNE, Monique**
  **F-92600 Asnières (FR)**
- **AMIRAL, Jean**
  **F-95130 Franconville (FR)**
- **MARTINOLI, Jean-Luc**
  **F-92390 Villeneuve-la-Garenne (FR)**

(74) Mandataire: **Clisci, Serge et al**
**S.A. FEDIT-LORIOT & AUTRES**
**CONSEILS EN PROPRIETE INDUSTRIELLE**
**38, Avenue Hoche**
**75008 Paris (FR)**

(56) Documents cités:
**WO-A-90/08321**    **WO-A-91/14787**
**FR-A- 2 190 424**    **FR-A- 2 680 519**

- **CHEMICAL ABSTRACTS, vol. 107, no. 11, 14 septembre 1987, Columbus, OH (US); G. TRONCHIN et al., p. 404, AN 93396**
- **CHEMICAL ABSTRACTS, vol. 108, no. 3, 18 janvier 1988, Columbus, OH (US); p. 342, AN 19126**
- **CHEMICAL ABSTRACTS, vol. 106, no. 21, 25 mai 1987, Columbus, OH (US); A. BOUALI et al., p. 513, AN 173914**

**Description**

## DOMAINE DE L'INVENTION

La présente invention a trait à un nouveau procédé pour mettre en évidence l'affinité du fibrinogène et de ses dérivés [i.e. la fibrine et les FDP qui comprennent les produits de dégradation du fibrinogène (FgDP) et les produits de dégradation de la fibrine (FnDP)] aux formes filamentaires de levures.

Ce procédé permet de distinguer les souches de levures affines qui fixent le fibrinogène et ses dérivés sur leurs formes filamentaires, notamment leurs formes germinatives qui comprennent les tubes germinatifs [en anglais : "germ-tubes"], des souches de levures non-affines qui ne fixent pas le fibrinogène et ses dérivés sur lesdites formes filamentaires.

L'invention concerne également l'utilisation de ce procédé dans le domaine de la détermination de l'affinité des levures, d'une part, et dans le domaine de la détermination des levures pathogènes, notamment en hémoculture, en particulier pour distinguer avantageusement les souches de levures qui sont pathogènes de celles qui sont non-pathogènes, d'autre part. Une telle utilisation est particulièrement intéressante dans le domaine thérapeutique en clinique humaine ou vétérinaire, dans le domaine agricole et dans le domaine alimentaire.

## ART ANTERIEUR

On sait que, parmi les agents infectieux qui ont été observés en pathologie, les levures jouent un rôle important et que leur influence est malheureusement en nette progression. On sait en particulier que les souches de **Candida albicans** sont celles que l'on rencontre le plus souvent dans les cas de candidoses et qu'elles représentent environ 50 % des cas des hémocultures positives ; on sait également que les souches de **Candida glabrata** (ancienne nomenclature : **Torulopsis glabrata)** sont devenues le second agent, par ordre d'importance, des septicémies, et que des souches de **Candida tropicalis** et **Candida krusei** sont à l'origine des autres cas de candidoses rencontrés en pathologie. On connait par ailleurs d'autres agents infectieux qui sont également fréquemment isolés et qui sont constitués par des souches de **Trichosporon** et de **Cryptococcus** (notamment les souches de **Cryptococcus neoformans)** dont l'incidence augmente de plus en plus chez les patients atteints du syndrome d'immuno-déficience acquise (SIDA).

Il se trouve que les diagnostics clinique et biologique des candidoses disséminées qui sont associées de façon croissante à l'immuno-dépression induite notamment par une thérapeutique ou une pathologie, ont une importance capitale sur le pronostic vital du malade.

Or, le diagnostic des candidoses profondes soulève de nombreux problèmes essentiellement liés à une absence de spécificité des signes cliniques, à l'insensibilité des hémocultures conventionnelles (négativité dans plus de 50 % des cas), à l'inaptitude de distinguer la colonisation de l'invasion, aux faibles sensibilité et fiabilité des méthodes sérologiques, et à la fugacité de la présence des antigènes solubles. En particulier, il se trouve que 5% des souches pathogènes de **Candida albicans** ne sont pas détectées par le test de blastèse après hémoculture ou tout autre isolement, dès lors que leurs blastospores ne donnent pas de tubes germinatifs dans les conditions usuelles de filamentation. Par ailleurs, les souches de levures conservées dans les collections et qui sont réputées pathogènes peuvent perdre leur caractère pathogène par repiquages successifs.

On sait par ailleurs, (a) des articles de P.A. MAISCH et al., *Infect. Immun.,* 1980, <u>27</u> (No 2), 650-656 et 1981, <u>31</u> (No 1), 92-97, (b) de l'article de L.P. SAMARANAYAKE et al., *Mycopathologia,* 1988, <u>102</u>, 135-138, (c) des articles de A. BOUALI, R. ROBERT, G. TRONCHIN et J.M. SENET, *J. Med. Vet. Mycol.,* 1986, <u>24</u>, 345-348 et J. *Gen. Microbiol.,* 1987, <u>133,</u> 545-551, et (d) de l'article de G. TRONCHIN, R. ROBERT, A. BOUALI et J.M. SENET, *Ann. Inst. Pasteur/ Microbiol.,* 1987, <u>138</u>, 177-187, que des souches de **Candida albicans** et, à un degré moindre d'autres espèces de levures appartenant notamment à l'ensemble des *Candida* peuvent fixer le fibrinogène (Fg) ou son dérivé, la fibrine (Fn).

Selon le premier article P.A. MAISCH et al., neuf isolats de levures ont été testés en ce qui concerne leur adhérence à des matrices fibrino-plaquettaires [en anglais : "fibrin-platelet matrices" (c'est-à-dire ici des caillots de fibrine-plaquettes, en anglais : "fibrin-platelet clots")], à savoir deux souches isolées de **Candida albicans,** une souche isolée de **Candida stellatoidea,** une souche isolée de **Candida tropicalis,** une souche isolée de **Candida parapsilosis,** une souche isolée de **Candida pseudotropicalis,** une souche isolée de **Saccharomyces cerevisiae,** une souche isolée de **Candida guillermondii** et une souche isolée de **Candida krusei,** et les résultats obtenus figurent dans le tableau 4 de la page 654. Ce tableau montre que (i) toutes les souches testées se fixent sur les matrices fibrino-plaquettaires, qu'elles soient présumées pathogènes (surtout les souches de **Candida albicans, Candida glabrata** et à la rigueur les souches de **Candida tropicalis, Candida parapsilosis** et **Candida pseudotropicalis)** ou réputées généralement peu pathogènes ou non-pathogènes (les souches de **Candida krusei, Saccharomyces cerevisiae** et **Candida guillermondii),** et (ii) la fixation ou adhérence est plus ou moins importante en fonction de la nature de la souche testée. Enfin cet article n'enseigne pas comment distinguer les souches de levures pathogènes des souches de levures non-pathogènes.

Selon l'article de L.P. SAMARANAYAKE et al., l'adhérence à des caillots de fibrine de six souches isolées de **Candida** a été étudiée, à savoir trois souches isolées de cavités orales de patients atteints de stomatite : **Candida albicans, Candida krusei** et **Candida guillermondii**,d'une part, et trois souches de référence provenant d'une collection : **Candida parapsilosis, Candida glabrata** et **Candida tropicalis,** d'autre part. Les résultats obtenus, consignés dans le tableau 1 de la page 136, conduisent aux mêmes conclusions que celles énumérées ci-dessus pour les résultats de P.A. MAISCH et al.

Par ailleurs, les techniques opératoires fournies par les articles de P.A. MAISCH et al., d'une part, et l'article de L.P. SAMARANAYAKE et al., d'autre part, à la différence de la solution technique préconisée par la présente invention, ne permettent pas de distinguer les souches de levures qui sont pathogènes des souches de levures qui ne sont pas pathogènes.

De plus, les modalités opératoires qui figurent dans lesdits articles de P.A. MAISCH et al., d'une part, et de L.P. SAMARANAYAKE et al., d'autre part, ne permettent pas de préciser clairement les formes des souches qui ont été testées, à savoir les formes levures proprement dites (i.e. les blastospores ou blastoconidies) qui ne conviennent pas dans le procédé de l'invention, et les formes filamentaires qui comprennent les formes germinatives (i.e. les tubes germinatifs) et les formes filamenteuses (i.e. le pseudomycélium et le mycélium) et qui elles conviennent selon le procédé de l'invention.

Les articles précités de A. BOUALI et al., qui sont plus précis en ce qui concerne la mention des formes testées, ont trait à l'adhérence du fibrinogène sur les blastospores, les levures et le mycélium de souches de **Candida albicans**. Selon le second article de A. BOUALI et al., (i) le fragment D (qui est un FDP particulier) a une plus grande affinité vis-à-vis du **Candida albicans** que le fragment E (qui est un autre FDP particulier), (ii) il existe une compétition au niveau de l'adhérence sur le mycélium entre le fibrinogène, le fragment D et le fragment E présents, et (iii) le fibrinogène présente une plus grande affinité pour le mycélium que pour les tubes germinatifs. Cependant, les deux articles de A. BOUALI et al., à la différence de la présente invention, n'enseignent pas l'utilisation de l'éventuelle affinité et de l'éventuelle adhérence de Fg, D et E dans le cadre d'un procédé permettant de distinguer les souches de levures affines, qu'elles appartiennent ou non à l'ensemble des **Candida,** qui fixent le fibrinogène et ses dérivés, des souches de levures non-affines, qui ne fixent pas le fibrinogène ni ses dits dérivés.

Selon l'article précité de G. TRONCHIN et al., on connaît en tant que réactif un matériau constitué par du fibrinogène fixé sur des billes de latex submicroniques ayant un diamètre de 300 nm (voir paragraphe intitulé "Reagents", page 178, en bas). Ce réactif Fg-latex a été utilisé dans une mesure impliquant la mise en oeuvre d'un microscope électronique à balayage [en anglais : "scanning electron microscope"] pcur localiser le site de l'adhérence sur le mycélium et les tubes germinatifs d'une souche de **Candida albicans** isolée d'un cas de septicémie. L'article de G. TRONCHIN et al., à la différence de la présente invention ne décrit ni ne suggère l'utilisation de l'affinité du réactif Fg-latex dans le cadre d'un procédé permettant de distinguer les levures affines des levures non-affines, d'une part, et de distinguer les souches de levures qui sont pathogènes de celles qui ne sont pas pathogènes, d'autre part.

En bref, les articles de A. BOUALI et al., d'une part, et l'article de G. TRONCHIN et al., d'autre part, visaient en d'autres termes la recherche et l'identification d'un facteur de fixation du fibrinogène, en abrégé FBF [de l'anglais : "fibrinogen-binding factor"], susceptible d'intervenir le cas échéant dans les mécanismes de patho-génicité, alors que selon l'invention on a voulu en premier lieu, distinguer les souches de levures qui fixent le fibrinogène et ses dérivés de celles qui ne les fixent pas, et en second lieu, distinguer les souches de levures qui sont pathogènes de celles qui ne sont pas pathogènes pour apprécier, notamment après prélèvement chez le patient, s'il y a eu colonisation ou invasion.

## BUT DE L'INVENTION

Selon l'invention on se propose de fournir une nouvelle solution technique pour mettre en évidence l'éventuelle affinité de Fg et de ses dérivés, Fn et FDP, vis-à-vis des formes filamentaires de levures. Cette nouvelle solution technique repose sur l'observation de l'adhérence ou de la non-adhérence de Fg et de ses dérivés sur les formes filamentaires de levures.

Selon cette nouvelle solution technique, on se propose de fournir un procédé permettant de distinguer les souches de levures affines, qu'elles appartiennent ou non à l'ensemble des **Candida,** qui fixent le fibrinogène et ses dérivés, des souches de levures non-affines, qui ne fixent pas le fibrinogène ni ses dits dérivés.

Selon un autre aspect de l'invention, on se propose de fournir un procédé permettant de distinguer les souches de levures qui sont pathogènes de celles qui ne sont pas pathogènes.

## OBJET DE L'INVENTION

La nouvelle solution technique selon l'invention, qui permet de pallier aux inconvénients précités de l'art antérieur, repose sur

- la multiplication des blastospores des souches de levures à tester,
- la filamentation desdites levures pour obtenir un volume relativement important de formes filamentaires essentiellement constituées de tubes germinatifs et/ou de mycélium,
- la mise en contact des formes filamentaires avec le fibrinogène, l'un de ses dérivés ou l'un de leurs mélanges, et
- l'appréciation de l'adhérence ou de la non-adhérence qui résulte de ladite mise en contact.

Selon un premier aspect de l'invention, on préconise un nouveau procédé pour mettre en évidence l'éventuelle affinité d'une substance (F) choisie parmi l'ensemble constitué par le fibrinogène (Fg), la fibrine (Fn), leurs produits de dégradation (FDP) et leurs mélanges, vis-à-vis des formes filamentaires de souches de levures, ledit procédé, qui comporte la mise en contact desdites formes filamentaires avec ladite substance (F) pour distinguer les souches de levures qui fixent ladite substance (F) de celles qui ne la fixent pas, étant caractérisé en ce qu'il comprend les étapes consistant à

(1°) cultiver (A) un échantillon contenant des levures à tester essentiellement sous forme de blastospores, susceptible de contenir au moins une substance pouvant interférer dans le mécanisme d'adhérence de ladite substance (F) sur les formes filamentaires desdites levures et convenablement dilué pour éliminer essentiellement toute interférence dans ledit mécanisme, dans (B) un milieu nutritif favorisant la multiplication des blastospores jusqu'à ce que l'on obtienne une population supérieure ou égale à $10^4$ levures/ml,
(2°) cultiver les blastospores de levures, ainsi obtenues, dans un milieu nutritif favorisant la filamentation,
(3°) mettre en contact les formes filamentaires, ainsi obtenues, avec une substance (F) choisie parmi l'ensemble constitué par le fibrinogène, la fibrine, les FDP et leurs mélanges, puis
(4°) apprécier l'adhérence ou la non-adhérence de ladite substance (F) sur lesdites formes filamentaires.

En variante, ce procédé peut ne comprendre que les étapes (2°) à (4°), l'étape (1°) pouvant être supprimée quand on dispose d'un échantillon contenant des levures, essentiellement constitué de blastospores à une concentration supérieure ou égale à $10^4$ levures/ml et mieux à une concentration supérieure ou égale à $10^5$ levures/ml, et essentiellement dépourvu ou appauvri en substance pouvant interférer dans le mécanisme d'adhérence de ladite substance (F) sur lesdites levures.

Le procédé selon l'invention pour mettre en évidence l'éventuelle affinité d'une souche de levure, est utile dans la détermination de l'affinité d'une substance (F) vis-à-vis des formes filamentaires de souches de levures dans le domaine thérapeutique en clinique humaine ou vétérinaire, dans le domaine agricole et dans le domaine alimentaire.

Selon un second aspect de l'invention, on préconise un procédé pour déterminer le caractère pathogène ou non-pathogène de souches de levures à tester, caractérisé en ce qu'il comprend

(1°) si nécessaire, la multiplication des blastospores d'un échantillon de souches de levures à tester jusqu'à ce que l'on obtienne une population d'au moins $10^4$ levures/ml et mieux une population supérieure ou égale à $10^5$ levures/ml,
(2°) la filamentation des blastospores,
(3°) l'incubation des formes filamentaires, ainsi obtenues, avec ladite substance (F) fixée sur un support inerte, de préférence des PDF globaux fixés sur un latex, et
(4°) l'observation du complexe éventuellement formé, de formule
    formes filamentaires-F-support, par agglutination.

Selon l'invention, on a trouvé de façon surprenante que les souches de levures, qui sont affines et fixent ladite substance (F) sont exclusivement ou essentiellement celles qui sont pathogènes.

On préconise enfin selon l'invention, un nécessaire, trousse ou kit de dosage ou de détermination, qui est caractérisé en ce qu'il comprend au moins un réactif substance (F)-support pour mettre en oeuvre la réaction :

$$\text{formes filamentaires} + \text{F-support} \rightarrow$$

$$\text{formes filamentaires-F-support}$$

La présente invention s'applique à l'ensemble des souches de levures qui filamentent. Elle s'applique également à l'ensemble des souches qui ne filamentent pas mais donne lieu à une pseudofilamentation dans laquelle les blastospores se recouvrent de petites sphérules, la configuration blastospore-sphérules se comportant ici comme un tube germinatif. En revanche, la présente invention ne s'applique pas aux levures qui ne donnent pas lieu à filamentation ni à pseudofilamentation.

## ABREVIATIONS ET NOTATIONS

Dans la présente description, on a utilisé les abréviations suivantes par commodité :

BL = blastospores (ou formes "levures" proprement dites)

D = fragment D, il s'agit d'un FDP particulier qui peut se présenter sous la forme "D monomère" (produit constitué d'une seule chaîne linéaire) ou sous la forme "D dimère" (produit essentiellement constitué de deux chaînes linéaires sensiblement parallèles)

E = fragment E, il s'agit d'un FDP particulier qui peut se présenter sous la forme "E monomère" (produit constitué d'une seule chaîne linéaire) ou sous la forme "E dimère" (produit essentiellement constitué de deux chaînes linéaires sensiblement parallèles)

EDTA = acide éthylènediaminotétraacétique

FBF = facteur de fixation du fibrinogène (ou facteur fixant le fibrinogène) [de l'anglais : "fibrinogen-binding factor"; autre nomenclature française : FFF]

FDP = produit de dégradation du fibrinogène ou de la fibrine [autre nomenclature française : PDF] ; l'expression FDP englobe d'une manière générale les produits de dégradation particuliers de fibrinogène/fibrine, à savoir les fragments D, E, X et Y, d'une part, et les produits de dégradation globaux de fibrinogène/fibrine, d'autre part ; sauf indication contraire, ici FDP désignera plutôt les produits de dégradation globaux.

Fg = fibrinogène

FgDP = produits de dégradation du fibrinogène [autre nomenclature française : PDFg]

Fn = fibrine

FnDP = produits de dégradation de la fibrine [autre nomenclature française : PDFn]

GT = tubes germinatifs [en anglais : "germ-tubes" ; autre nomenclature française : TG]

Leu = leucyle, L-Leu désignant le reste leucyle de configuration L

My = mycélium

OD = densité optique

PMy = pseudomycélium

pNA = paranitroanilino [ou $(4\text{-}NO_2)C_6H_4NH$]

SPS = polyméthanolsulfate de sodium

YNB = source d'azote commercialisée sous la nomenclature commerciale de "YEAST NITROGEN BASE"

Par ailleurs, les notations usuelles pour évaluer soit l'affinité soit l'agglutination, qui figurent dans plusieurs tableaux ci-après, sont les suivantes :

- = néant

+/- = néant et/ou faible

+ = faible

++ = moyenne

+++ = intense

++++ = très intense

## DESCRIPTION DETAILLEE DE L'INVENTION

Par "formes filamentaires" on entend ici l'ensemble constitué par les formes germinatives, qui sont essentiellement constituées par les GT, et les formes filamenteuses, qui sont essentiellement constituées par PMy et My.

D'une manière générale dans l'ensemble des levures qui filament, les BL sont approximativement des sphères ayant un diamètre moyen de 5 micromètres environ ; les GT sont approximativement constitués chacun d'un cylindre ayant une longueur moyenne de 15 micromètres environ et un diamètre moyen de micromètres environ et qui est lié par l'une de ses extrémités à une sphère ayant un diamètre moyen de 5 micromètres environ ; et My est approximativement constitué par des cylindres ayant chacun une longueur moyenne de 250 micromètres environ et un diamètre moyen de 5 micromètres environ. Par filamentation, les blastospores (ou blastoconidies) donnent les GT, puis les PMy et les My qui sont issus des GT.

Dans ce qui suit, on désigne, sauf indication contraire, par l'expression "souches de levures qui filament" l'ensemble des souches qui donnent des GT, PMy et My, d'une part, et des souches qui donnent lieu à une pseudofilamentation, d'autre part.

Lors de la mise au point de la présente invention on a (i) observé que la présence de Fg ou de FDP (i.e. FgDP et/ou FnDP) inhibe la multiplication des BL au cours de l'étape (1°), les BL ne fixant pas lesdits Fg et FDP, (ii) vérifié que Fn notamment sous la forme de caillot de fibrine présente une grande affinité vis-à-vis des BL comme indiqué par A. BOUALI et al., et constaté que la formation du complexe Fn-BL empêche la multiplication des BL au cours de l'étape (1°), (iii) observé que la présence de Fg ou de FDP (notamment FgDP et/ou FnDP) favorise la filamentation lors de l'obtention des formes filamentaires au cours de l'étape (2°), et (iv) découvert que les blastospores comportant des sphérules dans le cas des souches donnant lieu à une pseudofilamentation présentent une affinité vis-à-vis de la substance (F) analogue mais qualitativement plus faible que celle des tubes germinatifs des souches de levures qui filamentent.

Par suite, la présence de Fg ou de l'un de ses dérivés, soit au cours de l'étape (1°) dans le milieu de multiplication des BL, qui est constitué de l'échantillon de levures à tester, d'une part, et du milieu nutritif de multiplication, d'autre part, soit au cours de l'étape (2°) dans le milieu de filamentation qui est constitué au départ des BL qui ont déjà été multipliées et qui proviennent de l'échantillon de levures à tester, d'une part, et du milieu nutritif favorisant la filamentation, d'autre part, va fausser la mesure de l'adhérence (i.e. l'affinité) ou de la non-adhérence (i.e. l'absence d'affinité) de l'étape (4°), dès lors que à l'étape (3°) il y aura compétition entre (a) ladite substance (F) (i.e. le Fg ou l'un de ses dérivés, de préférence fixé sur un support inerte) que l'on ajoute à l'étape (3°), et (b) le Fg ou l'un au moins de ses dérivés déjà présent(s) à l'étape (1°) ou à l'étape (2°).

En conséquence, il convient selon l'invention que le milieu de multiplication des BL et le milieu de filamentation desdites BL soient dépourvus ou essentiellement appauvris en substance(s) pouvant interférer avec ladite substance (F) au cours de la réalisation de l'étape (3°).

Parmi les produits ou substances pouvant interférer avec ladite substance (F), on peut notamment citer le fibrinogène et ses dérivés, à savoir :

- la fibrine,
- les FDP particuliers que sont les fragments D, E, X et Y,
- les FDP globaux que sont les produits de dégradation FgDP et FnDP,

d'une part, et, le cas échéant,

- les protéines de l'inflammation,
- les composés antifongiques, et
- les anticorps anti(levure),

d'autre part.

Les substances interférentes énumérées ci-dessus ou certaines d'entre elles peuvent être présentes dans le sang total ou le plasma sanguin, d'une part, ainsi que dans les échantillons de levures qui ont été prélevés, d'autre part. Ainsi, le sang et le plasma contiennent en général du fibrinogène et le cas échéant un ou plusieurs de ses dérivés, et notamment dans les cas pathologiques des protéines de l'inflammation et/ou des anticorps anti(levure) [notamment des anticorps anti*(Candida)* dans les cas de candidoses] ; et les prélèvements de souches de levures de la famille des *Candida* peuvent contenir un composé antifongique tel que le cycloheximide qui inhibe la plupart des levures et en particulier les *Candida à l'exception des souches de Candida albicans* et de quelques souches de *Candida zeylanoides* (voir à cet effet la demande de brevet français No 90 03 726 du 23 mars 1990 de la demanderesse).

Dans la pratique de l'invention, on évite la présence de substance(s) interférente(s) dans le milieu nutritif de multiplication des BL et dans le milieu nutritif de filamentation des BL déjà multipliées grâce au choix approprié desdits milieux nutritifs. Par ailleurs, en fonction de l'origine de l'échantillon de levures à tester, il peut être impératif de diluer ledit échantillon pour l'appauvrir en substance(s) interférente(s).

Les milieux nutritifs de multiplication et de filamentation peuvent être liquides (i.e. milieux aqueux dits homogènes ou monophasiques) ou hétérogènes (i.e. milieux aqueux contenant une ou plusieurs matières solides et dits diphasiques). La multiplication et la filamentation peuvent être réalisées de façon aérobie ou anaérobie. On préfère selon l'invention les milieux aqueux monophasiques aérobies.

Par ailleurs, s'il est possible dans certains cas de procéder simultanément à la multiplication et à la filamentation, il est plus sûr et plus efficace de procéder à la multiplication (quand elle est nécessaire) puis à la filamentation. On recommande donc, notamment pour les hémocultures, de séparer les deux étapes : d'abord la multiplication puis la filamentation.

Une hémoculture consiste à prélever du sang (sang total ou plasma) et à faire pousser les microorganismes qu'il contient, à savoir les bactéries (hémoculture bactérienne) ou les levures (hémoculture fongique), la pousse des levures étant effectuée en présence d'un moyen antibactérien pour inhiber le développement des bactéries ou détruire lesdites bactéries dès lors qu'elles croissent généralement avant ou plus rapidement que les levures. Si nécessaire, les souches de levures qui ont poussé au cours de l'hémoculture fongique sont ensuite, soit isolées pour identification, soit identifiées sans isolation (quand il s'agit de *Candida)* selon l'enseignement de la demande française No 90 03 726 précitée.

On sait que l'hémoculture fongique (désignée ci-après "hémoculture" par commodité) est réalisée de façon standardisée sur un prélèvement de sang (sang total ou plasma) préalablement dilué à $10^{-1}$ dans un milieu nutritif de multiplication et/ou filamentation. Généralement un tel milieu est le milieu de Sabouraud ou le milieu trypticase-soja, additionné de SPS, de EDTA ou de citrate de sodium. Cependant, un tel milieu qui permet la croissance des champignons ne conduit pas toujours à l'obtention des formes filamentaires, notamment dans le cas de certaines souches de *Candida* par exemple.

Or, il se trouve que la dilution de $10^{-1}$ comporte une teneur en substance(s) interférente(s) trop importante. Selon l'invention, pour disposer dans le cadre d'une hémoculture d'un échantillon essentiellement appauvri en substance(s) interférente(s), il est critique d'utiliser à l'étape (1°) ou à l'étape (2°) un échantillon de sang préalablement dilué au moyen d'un milieu nutritif aqueux de multiplication ou de filamentation jusqu'à une dilution de $x^{-1}$, où x est un nombre supérieur ou égal à 50, c'est-à-dire par exemple une dilution allant de $50^{-1}$ à notamment $150^{-1}$ ou $200^{-1}$.

L'étape (1°) a pour objet la multiplication des BL jusqu'à ce que l'on obtienne une population supérieure ou égale à $10^4$ levures/ml (i.e. $10^4$ blastospores/ml) et de préférence une population supérieure ou égale à $10^5$ levures/ml (i.e. $10^5$ blastospores/ml). La durée de l'étape (1°) est en général inférieure ou égale à 48 h à une température comprise entre 25 et 40°C, de préférence à 37°C, lorsque ladite étape (1°) est mise en oeuvre.

Le milieu de multiplication des BL, qui est préféré selon l'invention, surtout pour les hémocultures, est constitué par un milieu aqueux, à savoir le milieu de Shadomy (qui renferme YNB, glucose et asparagine) enrichi en extrait de levure (1 à 4 g/l) et, si nécessaire, complémenté en saponine, urée et/ou antibactérien.

L'étape (1°) peut être supprimée quand on dispose déjà d'un échantillon de souches de levures ayant la population requise précitée et qui est dépourvu ou essentiellement appauvri en substance(s) interférente(s).

Si nécessaire, le milieu nutritif de multiplication des BL pourra comporter un moyen antibactérien pour inhiber ou éviter la croissance des éventuelles bactéries contenues dans l'échantillon de levures à tester (cette circonstance pourra se présenter dans le cas d'une hémoculture où ledit échantillon est un prélèvement de sang total ou de plasma), d'une part, et pourra comporter de préférence de l'urée pour permettre le développement de certaines souches de levures qui croissent difficilement, notamment les *Cryptococcus* et les *Candida glabrata.*

L'utilisation du milieu de Shadomy enrichi en extrait de levure, d'une part, et complémenté en saponine, urée et agent antibactérien (notamment la gentamycine et/ou l'ampicilline), d'autre part, assure le développement des champignons qui filamentent difficilement ou qui donnent lieu à une pseudofilamentation, à savoir (i) les souches de *Cryptococcus,* et de *Candida glabrata* précitées, (ii) les souches de *Rhodotorula* et de *Aspergillus,* et (iii) les souches particulières de *Candida albicans* présentes quelquefois dans des cas pathologiques mais non détectés par les hémocultures classiques en raison de leur faible développement.

Le milieu de multiplication de l'étape (1°) sera avantageusement un milieu aqueux aérobie.

Par ailleurs, dans le cadre d'une hémoculture, ledit milieu nutritif de multiplication des BL pourra avantageusement comporter une substance, qui (i) n'intervient pas sur la croissance des levures, (ii) est inerte vis-à-vis de l'affinité des formes filamentaires à la substance (F), et (iii) lyse les érythrocytes, les leucocytes et les macrophages pour libérer les vitamines et les facteurs de croissance contenus dans lesdits érythrocytes, d'une part, et pour libérer les éventuelles levures que fixent ou immobilisent lesdits leucocytes et macrophages, d'autre part. Parmi les substances lysantes de ce type qui conviennent on peut notamment citer les détergents tels que la saponine. Lorsque la saponine est utilisée, cette substance intervient à une concentration de 0,1 à 12 g/l (de préférence 0,2-0,4 g/l) dans le milieu nutritif.

De plus, toujours dans le cadre d'une hémoculture, il est important d'incorporer un agent anticoagulant approprié dans le milieu nutritif de multiplication des BL pour éviter la formation d'un caillot de Fn qui fausserait la réaction :

$$\text{formes filamentaires} + \text{F-support} \rightarrow$$

$$\text{formes filamentaires-F-support}$$

par le mécanisme de compétition précité.

Le moyen anticoagulant préféré est l'héparine. Le citrate de sodium et l'EDTA ne conviennent pas en tant que moyens anticoagulants, en ce sens qu'ils sont susceptibles d'inhiber le développement des formes filamentaires.

L'étape (2°) a pour objet la filamentation des BL pour obtenir les formes filamentaires. De façon pratique selon l'invention, la filamentation de l'étape (2°) est réalisée pendant une durée d'au moins 1 h à 25-40°C pour obtenir les formes filamentaires choisies parmi l'ensemble comprenant les formes germinatives et les formes filamenteuses dans le cas des souches de levures qui filamentent, ou des formes blastospores-sphérules dans le cas des souches de levures qui donnent lieu à une pseudofilamentation.

L'étape (2°) est réalisée dans un milieu aqueux, le pH optimal du milieu de filamentation se situant dans la gamme de 7,0 à 7,5 [c'est ce domaine de pH optimal qui sera avantageusement utilisé lors de l'étape (3°)].

Pour gagner du temps au cours de la mise en oeuvre du procédé selon l'invention, il suffit de cesser la filamentation dès que l'on a obtenu un volume suffisant de GT (ou de blastospores-sphérules) sans avoir besoin de poursuivre ladite filamentation jusqu'aux formes PMy et My. En d'autres termes, la filamentation de l'étape (2°) est réalisée pendant 1 à 4 h à 37°C pour obtenir principalement les formes germinatives, qui sont essentiellement constituées par les tubes germinatifs (ou dans le cadre des souches de levures donnant lieu à une pseudofilamentation, les blastospores-sphérules).

Parmi les milieux nutritifs qui conviennent pour la mise en oeuvre de la filamentation de l'étape (2°), on peut notamment citer les milieux de blastèse synthétiques et en particulier ceux utilisés de façon classique pour l'identification des souches isolées de *Candida.*

Le milieu nutritif que l'on préconise de préférence pour la filamentation de l'étape (2°) sera avantageusement le milieu de Shadomy (qui est un milieu aqueux contenant YNB, glucose et asparagine) non-enrichi en extrait de levure. En d'autres termes, le milieu nutritif préféré pour la filamentation, à savoir le milieu de Shadomy, sera différent du milieu nutritif préféré pour la multiplication, à savoir le milieu de Shadomy enrichi en extrait de levure, où ledit extrait de levure intervient à une concentration de 1 à 4 g/l dans le milieu de filamentation. Dans ce qui suit, on a désigné par l'expression "milieu de Shadomy enrichi", le milieu de Shadomy complémenté en extrait de levure à une concentration de 1-4 g/l (notamment à la concentration de 2 g/l).

Comme indiqué ci-dessus pour l'étape (1°), le milieu nutritif de multiplication pourra contenir, notamment dans le cadre d'une hémoculture :

- une substance lysant les érythrocytes, les leucocytes et les macrophages pour libérer les formes fongiques phagocytées par les leucocytes et les macrophages, d'une part, et les substances contenues dans les érythrocytes, d'autre part, ladite substance lysante devant bien entendu être inerte vis-à-vis de la croissance des levures à tester et de l'affinité des formes filamentaires desdites levures à tester à la substance (F), la substance lysante étant, comme indiqué ci-dessus, de préférence un détergent acceptable, et mieux la saponine ;
- un agent anticoagulant, de préférence l'héparine,
- de l'urée, et
- le cas échéant, un moyen antibactérien.

Dans le cadre d'une hémoculture, si l'échantillon de sang ou de plasma contient au départ une population importante de blastospores, l'on pourra en variante procéder à une dilution à $x^{-1}$, où x est un nombre supérieur ou égal à 50, de l'échantillon à tester contenant un anticoagulant, avec de préférence le milieu nutritif aqueux de multiplication, pour arriver à une population supérieure ou égale à $10^4$ ou mieux une population supérieure ou égale à $10^5$, et éviter ainsi la mise en oeuvre de l'étape (1°) en débutant directement par l'étape (2°).

En pratique, le milieu de filamentation de l'étape (2°) sera avantageusement un milieu aqueux aérobie.

Selon l'invention, la mise en contact de l'étape (3°) est une incubation pendant au moins 0,5 h à 37°C des formes filamentaires avec ladite substance (F) préalablement fixée sur un support inerte. La durée de l'étape (3°) sera en général comprise entre 0,5 h et 4 h à 37°C et avantageusement comprise entre 1 h et 3 h à 37°C. En pratique, une durée de 1 h à 37°C sera suffisante pour la plupart des souches de levures à tester qui filamentent, et une durée de 1 à 3 h à 37°C sera utilisée pour les souches qui donnent une pseudofilamentation telles que les *Candida glabrata.*

La substance (F) intervenant à l'étape (3°) est choisie parmi le fibrinogène et ses dérivés (Fn, D, E, FDP, notamment FgDP et FnDP).

De façon avantageuse, on utilisera des FDP globaux, à savoir les FgDP globaux et/ou les FnDP globaux.

Selon l'invention la substance (F) sera, pour la réalisation de l'étape (3°), fixée sur un support inerte, soit par liaison covalente, soit par adsorption. De façon avantageuse, ledit support inerte sera une substance polymérique telle que les latex.

Selon le meilleur mode de mise en oeuvre de l'invention, l'on fera appel à une substance (F) constituée par des PDF globaux fixés par covalence sur un support latex.

L'existence d'une affinité entre les formes filamentaires des souches de levures à tester et ladite substance (F) préalablement fixée sur un support inerte, se traduit par une agglutination.

Selon l'invention, l'étape (4°) comprend une opération de lecture choisie parmi l'ensemble constitué par

(a) l'observation de l'éventuelle adhérence au microscope de ladite substance (F) sur lesdites formes filamentaires de levures,
(b) l'observation de l'éventuelle agglutination du complexe
 formes filamentaires-F-support et,
(c) l'observation de l'éventuelle formation du complexe

formes filamentaires-F ou
formes filamentaires-F-support par un substrat enzymatique spécifique des enzymes de levures.

Ainsi, on dispose de trois solutions techniques pour apprécier l'éventuelle affinité de la substance (F) vis-à-vis des formes filamentaires de souches de levures.

La solution I, selon l'observation (a) ci-dessus, consiste à préparer des lames de microscope que l'on dispose sur la platine d'un microscope optique pour visualiser et/ou compter le nombre de particules F-latex fixées sur les GT (ou leurs analogues blastospores-sphérules) et, le cas échéant, les PMy et My. La solution I est essentiellement une observation de confirmation dès lors qu'elle est fastidieuse et exige beaucoup d'habitude ou expérience pour lire les lames de microscope.

La solution II, selon l'observation (b) ci-dessus, est une technique efficace pour déterminer l'affinité selon l'invention. L'observation (b) met en oeuvre l'agglutination d'un complexe

forme filamentaire-F-support et de préférence, le complexe
GT-FDP-latex.

Selon le choix du latex, la détermination peut être soit qualitative, soit quantitative. Avec des billes de latex ayant un diamètre supérieur à 1 micromètre et sur lesquelles est fixée la substance (F) on obtient une agglutination qualitative. Avec des billes de latex submicroniques ayant un diamètre inférieur à 1 micromètre et sur lesquelles est fixée la substance (F) [de préférence les billes de latex décrites dans la demande internationale PCT publiée WO-A-90/08 321] on obtient une agglutination quantitative.

Selon la solution technique II, on observe qualitativement (i) une agglutination à l'échelle macroscopique à l'oeil nu, ou (ii) une agglutination à l'échelle microscopique par mesure de la variation de OD. Lorsque le support est constitué par des billes de latex, il intervient en tant que moyen "amplifiant" le résultat de la réaction d'agglutination.

Avec des billes de latex submicroniques selon le document WO-A-90/08321 précité, on évalue quantitativement l'agglutination par la variation de OD.

La solution III, selon l'observation (c) ci-dessus, est une technique également efficace pour déterminer l'affinité selon l'invention. Elle repose sur un mécanisme enzymatique. En pratique, l'observation (c) met en oeuvre l'utilisation d'un substrat enzymatique spécifique des enzymes ayant une activité leucine-arylamidase. Par "enzyme ayant une activité leucine-arylamidase" on entend les enzymes qui clivent en -L-Leu-OH et H-R, les substrats aminoacides, dipeptides, tripeptides ou tétrapeptides se terminant au niveau de l'extrémité CO-terminale par un reste -L-Leu-R où Leu est le reste leucyle de l'extrémité CO-terminale et R est pNA ou un groupe chromogène analogue à pNA, tel que les restes p-nitroanilino comportant un substituant sur le noyau phényle (notamment Cl, Br, F, $CH_3$ ou $OCH_3$) et décrits dans EP-A-0 110 306. Les enzymes ayant une telle activité leucine-arylamidase sont des enzymes caractéristiques des levures se trouvant notamment dans les formes filamentaires.

Le substrat enzymatique que l'on préfère selon l'invention, est un substrat aminoacide, à savoir : H-L-Leu-pNA.

Dans ces conditions, la méthode de détermination par voie enzymatique comprend les stades consistant à

- faire appel à un support approprié (notamment une microplaque de titration ou un ensemble de cuvettes),
- revêtir ce support avec la substance (F),
- laver le support approprié pour éliminer la substance (F) non liée audit support approprié,
- mettre en contact ladite substance (F) fixée audit support approprié avec les formes filamentaires (de préférence les GT) des souches de levures à tester provenant de l'étape (2°) et incuber pendant au moins 0,5 h à 37°C (de préférence 1 à 2 h à 37°C),
- laver pour écarter les substances non fixées,
- révéler la réaction d'affinité, si elle s'est produite, au moyen d'un substrat enzymatique (H-L-Leu-pNA) spécifique des enzymes des levures, puis
- mesurer la variation de OD (notamment à 405 nm) pour quantifier l'activité enzymatique des formes filamentaires par rapport à un milieu témoin traité dans les mêmes conditions mais dépourvu de levures.

On a par ailleurs découvert de façon surprenante que dans la quasi-totalité des souches de levures testées, l'affinité F/formes filamentaires ne se manifeste que pour les souches de levures qui sont pathogènes. Par suite, l'affinité FgDP globaux/GT ou FnDP globaux/GT, d'une part, FgDP globaux/blastospores-sphérules ou FnDP globaux/blastospores-sphérules, d'autre part, constitue un moyen rapide, efficace et sûr pour déterminer si oui ou non des souches de levures à tester sont pathogènes.

Dans l'état actuel des connaissances, on présume que le mécanisme d'adhérence selon la réaction

formes filamentaires + F → formes filamentaires-F

EP 0 686 264 B1

est similaire aux mécanismes de la transformation des souches de levures non-pathogènes en souches pathogènes. Cependant, il s'agit là d'une théorie qui ne lie pas la Demanderesse. Il suffit dans le cadre de la présente invention de constater le résultat général que les souches de levures, dont les formes filamentaires sont affines vis-à-vis de la substance (F), sont essentiellement des souches pathogènes.

Cette découverte est particulièrement intéressante dans la détermination des levures pathogènes que l'on peut rencontrer en clinique humaine ou vétérinaire (notamment chez les animaux à sang chaud tels que les mammifères), dans le domaine agricole et dans le domaine alimentaire.

En particulier, il est très important de pouvoir apprécier (A) si des aliments sont ou non contaminés par des souches de levures pathogènes, (B) si des souches de levures destinées à (i) la fabrication des fromages à pâte persillée ou à croute fleurie, ou (ii) à la fabrication des moûts de bière sont ou non contaminées par des levures pathogènes, et (C) si des souches de levures devant être utilisées pour coloniser des récoltes (arachides, céréales et houblons notamment), afin d'empêcher le développement des levures néfastes vis-à-vis desdites récoltes, ne sont pas contaminées par des levures pathogènes. La protection des récoltes et des aliments par des levures est notamment décrite dans les documents FR-B-2 355 509 et FR-A-2 355 453.

En conséquence, on préconise l'utilisation de l'éventuelle affinité formes filamentaires/F pour déterminer le caractère pathogène ou non-pathogène de souches de levures à tester.

Comme indiqué plus haut, on préconise un procédé pour déterminer le caractère pathogène ou non-pathogène de souches de levures à tester, caractérisé en ce qu'il comprend

(1°) si nécessaire, la multiplication des blastospores d'un échantillon de souches de levures à tester jusqu'à ce que l'on obtienne une population d'au moins $10^4$ levures/ml et mieux une population supérieure ou égale à $10^5$ levures/ml,
(2°) la filamentation des blastospores,
(3°) l'incubation des formes filamentaires, ainsi obtenues, avec ladite substance (F) fixée sur un support inerte, de préférence des PDF globaux fixés sur un latex, et
(4°) l'observation du complexe éventuellement formé, de formule
formes filamentaires-F-support, par agglutination.

Selon ce procédé, la multiplication des blastospores à l'étape (1°) et/ou la filamentation desdites blastospores à l'étape (2°) par hémoculture sont effectuées dans un milieu liquide, de préférence aérobie, à partir d'un échantillon de sang ou plasma contenant des souches de levures à tester et préalablement dilué suivant une dilution de $x^{-1}$ où x est un nombre supérieur ou égal à 50, pour éliminer essentiellement toute interférence avec les autres substances contenues dans le sang ou le plasma et pouvant interférer dans le mécanisme d'adhérence de ladite substance (F) sur lesdites levures, les substances interférentes étant susceptibles de comprendre notamment Fg, Fn, FgDP et FnDP, d'une part, et les protéines de l'inflammation, les composés anti-fongiques qui ont pu être administrés, les anticorps *anti(Candida)*, d'autre part.

La solution technique préconisée par l'invention est particulièrement bien adaptée à la détermination du caractère pathogène ou non-pathogène des souches de levures qui filamentent pour donner des GT, PMy.et My.

## MEILLEUR MODE

Le meilleur mode de mise en oeuvre de l'invention pour les étapes (1°) à (4°) a été décrit ci-après, en ce qui concerne notamment les hémocultures.

Etape (1°)

on procède à la multiplication des blastospores contenues dans un échantillon de souches de levures à tester, notamment un échantillon de sang total ou de plasma sanguin, dans un milieu de multiplication des BL qui est aqueux, aérobie et constitué par un milieu de Shadomy enrichi en extrait de levure (où ledit extrait de levure est à une concentration de 1 à 4 g/l) et complémenté en saponine (0,2-0,4 g/l), en héparine, en urée et le cas échéant en moyen antibactérien, pendant une durée inférieure ou égale à 48 h à 37°C et à pH 7,0-7,5, l'échantillon de sang ou de plasma étant dilué au moyen dudit milieu de multiplication jusqu'à une dilution de $x^{-1}$ (où x est un nombre supérieur ou égal à 50), de façon à obtenir une population supérieure ou égale à $10^5$ levures/ml.

En variante, si on dispose d'un échantillon ayant une population convenable (i.e. une population supérieure ou égale à 5 x $10^6$ levures/ml), il suffit de diluer ledit échantillon au moyen du milieu de multiplication jusqu'à ladite dilution de $x^{-1}$.

Etape (2°)

On procède à la filamentation des blastospores obtenues selon l'étape (1°) ou sa variante sus-visée, dans un milieu aqueux aérobie constitué par le milieu de Shadomy non-enrichi et non-complémenté, pendant 1-4 h à 37°C et à pH 7,0-7,5, de façon à obtenir un volume suffisant de GT.

Etape (3°)

On incube un prélèvement du milieu contenant des GT obtenu à l'issue de l'étape (2°) avec un réactif (FDP-latex) constitué par des billes de latex revêtues de FDP globaux (FgDP globaux ou FnDP globaux), pendant au moins 1 h à 37°C et à pH 7,0-7,5.

Etape (4°)

On observe la formation du complexe résultant
GT-FDP-latex ou l'absence dudit complexe, par agglutination.

D'autres avantages et caractéristiques de l'invention seront mieux compris à la lecture de la description des exemples qui va suivre. Bien entendu, ces éléments ne sont nullement limitatifs mais sont donnés à titre d'illustration.

Dans ce qui suit, les exemples 1 à 5 ont trait à des essais en système dit purifié, et les exemples 6 à 9 ont trait à des essais en système plasmatique (sang total ou plasma).

**EXEMPLE 1 (comparatif)**

a) Protocole

On opère en système dit purifié. Différentes levures ayant poussé en 24 heures à 37°C sur milieu de Sabouraud sont ensemencées en milieu aqueux aérobie de filamentation à pH 7,0-7,5 de façon à obtenir des formes germinatives et/ou filamenteuses.

L'incubat est prélevé à différents temps et mis en présence, pendant 2 h à 37°C, de fibrine fixée de façon covalente sur une plaque de microtitration de façon à étudier l'affinité des levures à la fibrine.

Après lavage de la plaque, pour éliminer les levures non fixées, l'activité enzymatique leucine-arylamidase des levures fixées à la fibrine est révélée sur un substrat synthétique (H-L-Leu-pNA) et appréciée par la mesure de la OD à 405 nm.

b) Réaction d'affinité

Avec un milieu aqueux aérobie de filamentation constitué par le milieu synthétique "199" (commercialisé par l'Institut Pasteur et désigné ci-après "199 IP"), on a observé que Fn présente une affinité vis-à-vis des BL et des formes filamenteuses et que l'adhérence de Fn aux formes filamentaires (après incubation pendant 18 h à 37°C) est plus importante que celle de Fn aux BL (mesure à l'instant T = 0 h).

Les résultats consignés dans le tableau I ci-après, pour une souche de *Candida albicans,* confirment ceux donnés dans les articles A. BOUALI et al. précités.

Il convient de remarquer cependant que le milieu 199 IP n'est pas un milieu de filamentation approprié, ainsi que cela est démontré ci-après.

TABLEAU I

| Affinité à la fibrine en milieu synthétique 199 IP d'une souche de *Candida albicans* | | |
|---|---|---|
| Formes de la levure | Durée d'incubation (à 37°C et à pH 7,0-7,5) | OD (à 405 nm) |
| BL | T = 0 h | 0,057 |
| formes germinatives et filamenteuses | T = 18 h | 0,253 |

**EXEMPLE 2 (comparatif)**

On a testé différents milieux de développement de levures pour apprécier ceux qui favorisent la filamentation. Dans cette optique, on a mis à incuber 12 souches isolées de *Candida albicans* (connues pour filamenter facilement) dans chacun des milieux de développement (i.e. milieux de multiplication-filamentation) pendant 18 h à 37°C et à pH 7,0-7,5, puis on a observé au microscope la présence éventuelle des formes BL, GT, PMy et My.

Les résultats consignés dans le tableau II ci-après mettent en évidence que le milieu néopeptone ne favorise pas la filamentation, alors que les autres milieux, à savoir le milieu de Shadomy enrichi et éventuellement complémenté, le milieu 199 IP et le milieu plasma oxalaté favorisent ladite filamentation.

On verra plus loin que le supplément G (commercialisé par l'Institut Pasteur), le milieu 199 IP et le plasma oxalaté faussent la réaction d'affinité.

TABLEAU II

| Formes des levures selon les milieux de développement pour 12 souches de *Candida albicans* (incubation 18 h à 37°C à pH 7,0-7,5) | |
|---|---|
| Milieux | Formes des levures |
| néopeptone | rares formes BL bourgeonnantes rares GT |
| milieu 199 IP | très nombreuses formes BL bourgeonnantes et GT + PMy + My |
| milieu de Shadomy | nombreuses formes BL bourgeonnantes et GT + PMy + My |
| milieu de Shadomy + extrait de levure (2 g/l) | très nombreuses formes BL bourgeonnantes et GT + PMy + My |
| milieu de Shadomy + extrait de levure (4 g/l) + urée (2 g/l) + ampicilline | très nombreuses formes BL bourgeonnantes et GT + PMy + My |
| milieu de Shadomy + supplément G (IP) | très nombreuses formes BL bourgeonnantes et GT + PMy + My |
| plasma oxalaté | très nombreuses formes BL bourgeonnantes et GT + PMy + My |

## EXEMPLE 3 (comparatif)

On a apprécié l'affinité de différentes substances (F) vis-à-vis des formes filamentaires de plusieurs souches de levures. Dans cette optique, des souches isolées ont été mises à multiplier dans le milieu de Shadomy enrichi (extrait de levure à 2 g/l) pendant 18 h à 37°C et à pH 7,0-7,5, puis à filamenter dans le milieu de Shadomy normal (i.e. non-enrichi et non-complémenté) pendant 1-3 h à 37°C et à pH 7,0-7,5. A l'issue de la filamentation, des prélèvements du milieu de filamentation résultant ont été déposés dans les cupules de plaques de microtitration revêtues par liaison covalente de fibrinogène (Fg), de fibrine (Fn) obtenue par action de la thrombine sur le fibrinogène, de fragment D purifié (D), de FgDP obtenus par action de la plasmine sur le fibrinogène, et de FnDP obtenus par action de la thrombine puis d'un activateur du plasminogène tel que l'urokinase. Les formes filamentaires obtenues, à partir de souches pathogènes de *Candida albicans* et de *Candida tropicalis,* et de souches non-pathogènes de *Candida krusei* et de *Candida glabrata* (toutes ces souches ayant été isolées en clinique humaine), ont été mises en contact avec Fg, Fn, D, FgDP ou respectivement FnDP pendant 2 h à 37°C et à pH 7,0-7,5.

La mesure de l'activité enzymatique leucine-arylamidase a été déterminée sur un substrat synthétique (H-L-Leu-pNA) par la détermination de la variation de OD à 405 nm, comme indiqué à l'exemple 1 ci-dessus.

Les résultats obtenus sont consignés dans le tableau III ci-après où sont données lesdites variations de OD à 405 nm.

TABLEAU III

| Variation de OD à 405 nm | | | | | |
|---|---|---|---|---|---|
| Souches | Fg | Fn | D | FgDP | FnDP |
| *Candida albicans* | 0,10 latence | 0,23 | 0,01 | 0,22 | 0,19 |
| *Candida tropicalis* | 0,61 latence | 0,64 | 0,60 | 0,89 | 0,89 |
| *Candida krusei* | 0 | 0 | 0 | 0 | 0 |

TABLEAU III   (suite)

| Variation de OD à 405 nm | | | | | |
|---|---|---|---|---|---|
| Souches | Fg | Fn | D | FgDP | FnDP |
| *Candida glabrata* | 0 | 0 | 0 | 0 | 0 |

**EXEMPLE 4 (comparatif)**

(a) Première série d'essais

on a évalué l'affinité de Fg et de ses dérivés (Fn, D, E, FgDP et FnDP) vis-à-vis des formes filamentaires de diverses levures, selon les modalités opératoires décrites à l'exemple 3 ci-dessus, en fonction de l'inoculum.

On a constaté que l'affinité vis-à-vis desdites formes filamentaires se manifeste effectivement pour les souches de levures pathogènes quand l'inoculum soumis à la filamentation a une population supérieure ou égale à $10^4$ levures/ml et mieux une population supérieure ou égale à $10^5$ levures/ml.

(b) Seconde série d'essais

On a évalué l'influence du milieu de développement de levures sur ladite affinité. Dans ce but, on a procédé au développement (i.e. multiplication et filamentation simultanées dans le même milieu) de souches de levures dans divers milieux de développement pendant 24 h à 37°C et à pH 7,0-7,5 ; ensuite on a déposé des prélèvements de chaque milieu de développement résultant dans des cupules de plaques de microtitration revêtues de Fg par liaison covalente, et laissé incuber pendant 2 h à 37°C et à pH 7,0-7,5 ; on a enfin mesuré l'activité enzymatique sur un substrat synthétique (H-L-Leu-pNA) par la détermination de la variation de OD (notamment à 405 nm) pour apprécier l'éventuelle affinité, comme indiqué à l'exemple 1 ci-dessus.

Les résultats obtenus avec des souches pathogènes de *Candida albicans* et de *Candida tropicalis* isolées en clinique humaine sont consignés dans le tableau IV ci-après.

TABLEAU IV

| Milieux | *C. albicans* | *C. tropicalis* |
|---|---|---|
| milieu 199 IP | 0,002 | 0,02 |
| milieu de Shadomy | 0,21 | 0,83 |
| milieu de Shadomy + extrait de levure | 0,32 | 1,1 |
| milieu de Shadomy + supplément G | 0 | 0 |
| plasma oxalaté | 0 | 0 |

Les résultats du tableau IV montrent que le milieu 199 IP, le milieu de Shadomy contenant le supplément G et le plasma oxalaté ne conviennent pas pour la mise en oeuvre de l'invention. Le supplément G, qui contient Fn, empêche l'adhérence des formes filamentaires sur le Fg des plaques de microtitration eu égard au mécanisme de compétition précité. De même, le milieu 199 IP fausse le résultat de la mesure d'affinité par ledit mécanisme de compétition. Le plasma oxalaté inhibe la filamentation des BL.

Le milieu de Shadomy normal (i.e. non-enrichi et non-complémenté) convient en tant que milieu de développement (i.e. milieu de multiplication-filamentation). En revanche, le milieu de Shadomy enrichi (extrait de levure à 1-4 g/l) utilisé en tant que milieu de développement est susceptible de fausser la réaction d'affinité, en ce sens que le Fg contenu dans l'extrait de levure, qui favorise la multiplication, peut se fixer sur les formes filamentaires lors de la filamentation , et les complexes

formes filamentaires-Fg formés lors de ladite filamentation peuvent se fixer sur le Fg de la plaque de microtitration. Par suite, il est plutôt préférable d'utiliser le milieu de Shadomy enrichi lors de la multiplication puis le milieu de Shadomy normal lors de la filamentation.

**EXEMPLE 5**

On procède à la multiplication des BL de diverses souches de levures de référence (provenant de collections reconnues) et d'isolats (provenant de prélèvements cliniques), dans un milieu aqueux aérobie constitué du milieu de Shadomy enrichi (extrait de levure à 2 g/l) et complémenté en urée (2 g/l), saponine (0,2 g/l), héparine et ampicilline, pendant 18 h à 37°C et à pH 7,0-7,5 ; on procède ensuite à la filamentation des blastospores ainsi obtenues, dans un milieu aqueux aérobie constitué par le milieu de Shadomy normal, pendant 1-4 h à 37°C et à pH 7,0-7,5, pour obtenir les formes GT (ou leurs analogues blastospores-sphérules), les souches soumises à la filamentation (ou pseudofilamentation) ayant une population de $10^5$ à $10^6$ levures/ml. On introduit ensuite le milieu de filamentation ainsi obtenu qui contient des GT (ou leurs dits analogues) dans les cupules de plaques de microtitration revêtues de façon covalente de Fg, puis laisse incuber pendant 1-3 h à 37°C. On évalue ensuite l'affinité révélée par le substrat H-L-Leu-pNA par la variation de OD à 405 nm. Les résultats obtenus sont consignés dans les tableaux Va (souches de référence) et Vb (souches d'isolats) ci-après.

TABLEAU Va

| (Souches de référence) | | |
|---|---|---|
| Espèces | Nombre de souches | |
| | testées | affines |
| *Candida albicans* | 2 (a) | 2 |
| *Candida albicans* | 2 (b) | 0 |
| *Candida tropicalis* | 1 (a) | 0 |
| *Candida tropicalis* | 2 (b) | 0 |
| *Candida krusei* | 2 (a) | 1 |
| *Candida krusei* | 1 (b) | 0 |
| *Candida glabrata* | 1 (a) | 1 |
| *Candida glabrata* | 1 (b) | 0 |
| *Candida parapsilosis* | 2 (b) | 0 |
| *Rhodotorula* | 2 (a) | 1 |
| *Rhodotorula* | 2 (b) | 0 |
| *Trichosporum* | 1 (a) | 1 |
| *Cryptococcus* | 1 (a) | 1 |
| *Cryptococcus* | 1 (b) | 0 |
| *Saccharomyces* | 2 (b) | 0 |
| *Geotrichum* | 1 (a) | 1 |
| *Aspergillus* | 1 (a) | 1 |
| Notes<br>(a) : souche(s) réputée(s) pathogène(s)<br>(b) : souche(s) réputée(s) non-pathogène(s) | | |

TABLEAU Vb

| (souches d'isolats) | | |
|---|---|---|
| Espèces | Nombre de souches | |
| | testées | affines |
| *Candida albicans* | 4 (c) | 4 |
| *Candida albicans* | 7 (d) | 0 |
| *Candida tropicalis* | 5 (c) | 5 |
| *Candida tropicalis* | 4 (d) | 0 |
| *Candida krusei* | 2 (c) | 2 |
| *Candida krusei* | 8 (d) | 0 |
| *Candida glabrata* | 2 (c) | 2 |
| *Candida glabrata* | 13 (d) | 0 |
| *Candida parapsilosis* | 1 (d) | 0 |
| Notes<br>(c) : souche(s) reconnue(s) pathogène(s)<br>(d) : souche(s) reconnue(s) non-pathogène(s) | | |

Les résultats des tableaux Va et surtout Vb mettent en évidence l'intérêt de l'invention pour distinguer les souches de levures qui sont pathogènes de celles qui ne le sont pas. Le tableau Va confirme par ailleurs que des souches de référence réputées pathogènes perdent leur pathogénicité par repiquages successsifs.

**EXEMPLE 6 (comparatif)**

(a) Protocole

On fait filamenter dans le milieu de Shadomy normal, pendant 4 h à 37°C et à pH 7,0-7,5, des souches de levures ayant une population supérieure ou égale à $10^5$ levures/ml et provenant d'un prélèvement de sang (contenant de la fibrine), selon une technique d'hémoculture après dilution à $10^{-1}$ du prélèvement sanguin dans un milieu de multiplication constitué par le milieu de Shadomy enrichi (extrait de levure à 2 g/l) et complémenté en urée (2 g/l).

On procède ensuite à la mise en évidence de l'éventuelle affinité en mettant en contact, pendant 1 h à 37°C, des prélèvements du milieu de filamentation résultant avec un réactif fixé par liaison covalente à des billes de latex, à savoir le réactif FDP-latex.

(b) Résultats

Les résultats obtenus avec des souches de *Candida* qui sont consignés dans le tableau VI ci-après, mettent en évidence la nécessité de faire filamenter les levures en milieu plasmatique après avoir incorporé un agent anticoagulant. Dans cet exemple, la présence de Fn sous la forme d'un caillot qui s'est formé fausse la mesure de l'affinité du réactif FDP-latex du fait du mécanisme de compétition entre Fn et FDP-latex.

TABLEAU VI

| Affinité de *Candida* à FDP-latex en présence d'un caillot de Fn | | |
|---|---|---|
| Milieux | Pousse (4 h à 37°C) | Affinité (FDP-latex 1 h à 37°C) |
| plasma citraté + CaCl$_2$) <br> . caillot Fn | +++ <br> (*candida* fixés à Fn) | - |
| . sérum | (absence de *Candida*) | - |
| plasma citraté | +++ | - |
| plasma hépariné | ++++ | - |
| sang hépariné + saponine (10 g/l) | ++++ | - |

**EXEMPLE 7 (comparatif)**

On a étudié l'influence de la dilution du système plasmatique sur l'évaluation de l'affinité de souches de *Candida* qui filamentent normalement (en donnant les GT, PMy et My).

Dans ce but, le prélèvement plasmatique (plasma sanguin ou sang total) a été additionné d'un moyen anti-coagulant dans la minute qui suit la prise de sang, puis dilué ensuite au moyen du milieu de Shadomy enrichi (extrait de levure à 2 g/l) et complémenté en saponine (10 g/l), urée (2 g/l) et ampicilline.

La filamentation a été réalisée dans le milieu de Shadomy normal, pendant 4 h à 37°C et à pH 7,0-7,5, à partir d'une population initiale de souches de levures de $10^5$ à $10^6$ levures/ml.

Pour apprécier l'affinité des formes filamentaires essentiellement constituées ici de GT, on a mis en contact des prélèvements du milieu de filamentation (tel qu'obtenu après culture pendant 4 h à 37°C et à pH 7,0-7,5) avec le réactif FDP-latex de l'exemple 6 pendant 1 h à 37°C et à pH 7,0-7,5.

La lecture de l'éventuelle affinité a été effectuée par la détermination de l'agglutination et a été vérifiée par examen microscopique.

Les résultats qui ont été obtenus sont consignés dans le tableau VII ci-après.

On constate que l'affinité n'est pas mise en évidence pour les formes levures proprement dites, c'est-à-dire les blastospores. En revanche, l'affinité est spécifique pour les formes filamentaires des levures testées et plus principalement les GT desdites levures.

Par ailleurs, il est important de séparer l'étape de multiplication de l'étape de filamentation pour éviter de fausser la détermination de l'affinité lors de la mise en contact de portions du milieu résultant de la filamentation avec le réactif FDP-latex.

TABLEAU VII

| Influence de la dilution | | | | | | |
|---|---|---|---|---|---|---|
| Système plasmatique | Dilution | | | | | |
| | $2^{-1}$ | $5^{-1}$ | $10^{-1}$ | $20^{-1}$ | $25^{-1}$ | $50^{-1}$ |
| plasma hépariné | | | | | | |
| (1) | - | - | - | + | + | +++ |
| (2) | NR | NR | NR | R+NR | R+NR | R |
| sang hépariné + saponine | | | | | | |
| (1) | - | - | - | + | + | +++ |
| (2) | NR | NR | NR | R+NR | R+NR | R |
| Notes<br>(1) : agglutination latex<br>(2) : examen microscopique<br>NR : les formes filamentaires (essentiellement GT) ne se pas recouvertes par le réactif FDP-latex (absence d'adhérence dudit réactif)<br>R : les formes filamentaires (essentiellement GT) sont recouvertes par le réactif FDP-latex (adhérence dudit réactif) | | | | | | |

## EXEMPLE 8 (comparatif)

On a étudié l'influence de la concentration des souches de levures dans le système plasmatique sur l'évaluation de l'affinité de souches de *Candida* qui filamentent normalement (en donnant les GT, PMy et My).

Dans ce but, le prélèvement plasmatique (sang total) a été additionné d'héparine en tant que moyen anti-coagulant dans la minute qui suit la prise de sang, puis dilué ensuite à $50^{-1}$ au moyen du milieu de Shadomy enrichi (extrait de levure à 2 g/l) et complémenté en saponine (10 g/l), urée (2 g/l) et ampicilline.

La filamentation a été réalisée ensuite (avec ou sans multiplication dans le milieu de Shadomy normal pendant 4 h à 37°C et à pH 7,0-7,5, à partir d'une population initiale de souches de levures ayant une population de $10^2$ à $10^6$ levures/ml.

Pour apprécier l'affinité des formes filamentaires essentiellement constituées ici de GT, on a mis en contact des prélèvements du milieu de filamentation (tel qu'obtenu après culture pendant 4 h à 37°C et à pH 7,0-7,5) avec le réactif FDP-latex de l'exemple 6 pendant 1 h à 37°C.

La lecture de l'éventuelle affinité a été effectuée par la détermination de l'agglutination et a été vérifiée par examen microscopique, comme indiqué à l'exemple 7 ci-dessus.

Les résultats qui ont été obtenus avec des souches de *Candida* sont consignés dans le tableau VIII ci-après. On constate, comme à l'exemple 7, que l'affinité n'est pas mise en évidence pour les formes levures proprement dites, c'est-à-dire les blastospores. En revanche, l'affinité est spécifique pour les formes filamentaires des levures testées et plus principalement les GT desdites levures. L'intérêt de séparer l'étape de multiplication (si elle est nécessaire) de l'étape de filamentation est également confirmé.

TABLEAU VIII

| Influence de la concentration des levures | | | | | |
|---|---|---|---|---|---|
| Croissance à 37°C | Population (levures/ml) | | | | |
| | $10^6$ | $10^5$ | $10^4$ | $10^3$ | $10^2$ |
| T = 0 h (BL) | | | | | |
| (1) | - | - | - | - | - |
| (2) | NR | NR | NR | NR | NR |
| T = 4 h (GT) | | | | | |

TABLEAU VIII   (suite)

| Influence de la concentration des levures | | | | | |
|---|---|---|---|---|---|
| Croissance à 37°C | Population (levures/ml) | | | | |
| | $10^6$ | $10^5$ | $10^4$ | $10^3$ | $10^2$ |
| (1) | + | +/- | - | - | - |
| (2) | R+NR | R+NR | NR | NR | NR |
| T = 20 h (BL) et 4 h (GT) | | | | | |
| (1) | +++ | +++ | ++ | + | +/- |
| (2) | R | R | R | R+NR | R+NR |
| Notes<br>(1) : agglutination (évaluée après 1h à 37°C)<br>(2) : examen microscopique effectué parallèlement à l'évaluation de l'agglutination<br>NR : les formes filamentaires (essentiellement GT) ne se pas recouvertes par le réactif FDP-latex (absence d'adhérence dudit réactif)<br>R : les formes filamentaires (essentiellement GT) sont recouvertes par le réactif FDP-latex (adhérence dudit réactif) | | | | | |

## EXEMPLE 9

On a mesuré l'affinité de diverses souches de levures en système plasmatique (sang total hépariné) qui ont été soumises d'abord à une multiplication pendant 24 h à 37°C, après dilution à $50^{-1}$ dans le milieu de Shadomy enrichi (extrait de levure à 2 g/l) et complémenté en urée (2 g/l), saponine (0,2 g/l) et antibactérien (ampicilline), pour multiplier les BL jusqu'à une population de $10^5$ à $10^6$ ; puis à une filamentation ou pseudofilamentation dans le milieu de Shadomy normal pendant 1-3 h à 37°C et à pH 7,0-7,5.

L'évaluation de l'affinité a été effectuée en mettant en contact pendant 1 h à 37°C des portions du milieu de filamentation résultant avec le réactif FDP-latex de l'exemple 6.

Les résultats qui ont été obtenus sont consignés dans le tableau IX ci-après.

TABLEAU IX

| Affinité de souches de levures en sang total hépariné | | |
|---|---|---|
| Espèces | Nombre de souches | |
| | testées | affines |
| *Candida albicans* | 6 (a) | 6 |
| *Candida albicans* | 1 (b) | 0 |
| *Candida tropicalis* | 4 (a) | 4 |
| *Candida tropicalis* | 1 (b) | 0 |
| *Candida glabrata* | 1 (a) | 1 |
| *Candida glabrata* | 1 (b) | 0 |
| *Cryptococcus neoformans* | 1 (b) | 0 |
| *Cryptococcus neoformans* | 1 (a) | 1 |
| *Cryptococcus laurentii* | 1 (b) | 0 |
| Notes<br>(a) : souche(s) reconnue(s) pathogène(s)<br>(b) : souche(s) reconnue(s) non-pathogène(s) | | |

Les résultats du tableau IX mettent en évidence l'intérêt considérable de la solution technique selon l'invention pour distinguer, en milieu plasmatique, les souches de levures pathogènes des souches de levures qui ne sont pas pathogènes.

Selon les modalités opératoires décrites à l'exemple 9 ci-dessus, mais en utilisant

(i) un milieu de multiplication diphasique et non monophasique,

(ii) un milieu de filamentation diphasique et non monophasique, et

(iii) un milieu de multiplication diphasique et non monophasique, puis un milieu de filamentation diphasique et non monophasique,

on obtient des résultats analogues à ceux consignés dans le tableau IX ci-dessus.

## Revendications

1.  Procédé pour mettre en évidence l'éventuelle affinité d'une substance (F) choisie parmi l'ensemble constitué par le fibrinogène (Fg), la fibrine (Fn), leurs produits de dégradation (FDP) et leurs mélanges, vis-à-vis des formes filamentaires de souches de levures, ledit procédé, qui comporte la mise en contact desdites formes filamentaires avec ladite substance (F) pour distinguer les souches de levures qui fixent ladite substance (F) de celles qui ne la fixent pas, étant caractérisé en ce qu'il comprend les étapes consistant à

    (1°) cultiver (A) un échantillon contenant des levures à tester essentiellement sous forme de blastospores, susceptible de contenir au moins une substance pouvant interférer dans le mécanisme d'adhérence de ladite substance (F) sur lesdites levures et convenablement dilué pour éliminer essentiellement toute interférence dans ledit mécanisme, dans (B) un milieu nutritif favorisant la multiplication des blastospores jusqu'à ce que l'on obtienne une population supérieure ou égale à $10^4$ levures/ml,

    (2°) cultiver les blastospores de levures, ainsi obtenues, dans un milieu nutritif favorisant la filamentation,

    (3°) mettre en contact les formes filamentaires, ainsi obtenues, avec une substance (F) choisie parmi l'ensemble constitué par le fibrinogène, la fibrine, les FDP et leurs mélanges, puis

    (4°) apprécier l'adhérence ou la non-adhérence de ladite substance (F) sur lesdites formes filamentaires.

2.  Procédé suivant la revendication 1, caractérisé en ce qu'il comprend les étapes (2°) à (4°), l'étape (1°) pouvant être supprimée quand on dispose d'un échantillon contenant des levures, essentiellement constitué de blastospores à une concentration supérieure ou égale à $10^4$ levures/ml et mieux supérieure ou égale à $10^5$ levures/ml, et essentiellement dépourvu ou appauvri en substance pouvant interférer dans le mécanisme d'adhérence de ladite substance (F) sur lesdites levures.

3.  Procédé suivant l'une quelconque des revendications 1 et 2, caractérisé en ce que la filamentation de l'étape (2°) est réalisée pendant une durée d'au moins 1h à 25-40° C pour obtenir les formes filamentaires choisies parmi l'ensemble comprenant les formes germinatives et les formes filamenteuses dans le cas des souches de levures qui filamentent, ou des formes blastospores-sphérules dans le cas des souches de levures qui donnent lieu à une pseudofilamentation.

4.  Procédé suivant l'une quelconque des revendications 1 et 2, caractérisé en ce que la filamentation de l'étape (2°) est réalisée pendant 1 à 4 h à 37°C pour obtenir principalement les formes germinatives, qui sont essentiellement constituées par les tubes germinatifs ou leurs analogues blastospores-sphérules.

5.  Procédé suivant l'une quelconque des revendications 1 et 2, caractérisé en ce que la mise en contact de l'étape (3°) est une incubation pendant au moins 0,5 h à 37°C des formes filamentaires avec ladite substance (F) préalablement fixée sur un support inerte.

6.  Procédé suivant la revendication 5, caractérisé en ce que ledit support inerte est un latex.

7.  Procédé suivant la revendication 5, caractérisé en ce que ladite substance (F) est choisie parmi l'ensemble des PDF du fibrinogène (FgDP) et de la fibrine (FnDP).

8.  Procédé suivant la revendication 5, caractérisé en ce que ladite substance (F) est choisie parmi l'ensemble des PDF globaux fixés sur un support latex.

9.  Procédé suivant l'une quelconque des revendications 1, 2 et 5, caractérisé en ce que l'étape (4°) comprend une opération de lecture choisie parmi l'ensemble constitué par

    (a) l'observation de l'éventuelle adhérence au microscope de ladite substance (F) sur lesdites formes filamentaires de levures,

(b) l'observation de l'éventuelle agglutination du complexe

formes filamentaires-F-support et,

(c) l'observation de l'éventuelle formation du complexe

formes filamentaires-F ou

formes filamentaires-F-support par un substrat enzymatique spécifique des enzymes de levures.

10. Procédé suivant la revendication 9, caractérisé en ce que l'observation (c) met en oeuvre un substrat enzymatique spécifique des enzymes ayant une activité leucine-arylamidase.

11. procédé suivant la revendication 10, caractérisé en ce que ledit substrat enzymatique est H-L-Leu-pNA.

12. Procédé suivant la revendication 9, caractérisé en ce que l'observation (b) met en oeuvre l'agglutination du complexe

GT-FDP-latex où GT représente les tubes germinatifs ou leurs analogues blastospores-sphérules.

13. Utilisation du procédé suivant l'une quelconque des revendications -1 à 12 pour la détermination de l'affinité d'une substance (F) vis-à-vis des formes filamentaires de souches de levures dans le domaine thérapeutique en clinique humaine ou vétérinaire, dans le domaine agricole et dans le domaine alimentaire.

14. Utilisation suivant la revendication 13, pour déterminer le caractère pathogène ou non-pathogène de souches de levures à tester.

15. Procédé pour déterminer le caractère pathogène ou non-pathogène de souches de levures à tester, conforme à l'utilisation suivant la revendication 14, caractérisé en ce qu'il comprend

(1°) si nécessaire, la multiplication des blastospores d'un échantillon de souches de levures à tester jusqu'à ce que l'on obtienne une population d'au moins $10^4$ levures/ml et mieux une population supérieure ou égale à $10^5$ levures/ml,

(2°) la filamentation des blastospores,

(3°) l'incubation des formes filamentaires, ainsi obtenues, avec ladite substance (F) fixée sur un support inerte, de préférence des PDF globaux fixés sur un latex, et

(4°) l'observation du complexe éventuellement formé, de formule

formes filamentaires-F-support, par agglutination.

16. Procédé suivant la revendication 15, caractérisé en ce que la multiplication des blastospores à l'étape (1°) ou la filamentation desdites blastospores à l'étape (2°) est effectuée par hémoculture dans un milieu liquide de préférence aérobie, à partir d'un échantillon de sang ou plasma contenant des souches de levures à tester et préalablement dilué suivant une dilution de $x^{-1}$, où x est un nombre supérieur ou égal à 50, pour éliminer essentiellement toute interférence avec les autres substances contenues dans le plasma et pouvant interférer dans le mécanisme d'adhérence de ladite substance (F) sur lesdites levures, les substances interférentes étant susceptibles de comprendre notamment Fg, Fn, FgDP et FnDP, d'une part, et les protéines de l'inflammation, les composés antifongiques qui ont pu être administrés, les anticorps anti*(Candida)*, d'autre part.

Claims

1. A method for revealing the possible affinity of a substance (F) selected from the group consisting of fibrinogen (Fg), fibrin (Fn), their degradation products (FDP) and mixtures thereof, for the filamentous forms of yeast strains, said process, which comprises bringing said filamentous forms into contact with said substance (F) in order to distinguish the yeast strains which bind said substance (F) from those which do not bind it, being characterised in that it comprises the following steps:

(1) cultivating (A) a sample containing the yeasts to be tested mainly in the form of blastospores, which sample can contain at least one substance which can interfere with the adherence mechanism of said substance (F) to the filamentous forms of said yeasts and which is suitably diluted to eliminate substantially all interference with said mechanism, in (B) a nutrient medium which promotes the multiplication of the blastospores in order to obtain a population higher than or equal to $10^4$ yeasts/ml,

(2) cultivating the yeast blastospores obtained in a nutrient medium which promotes the filamentation,
(3) bringing the filamentous forms thus obtained into contact with a substance (F) selected from the group consisting of fibrinogen, fibrin, FDP and mixtures thereof, then
(4) evaluating the adherence or non-adherence of said substance (F) to said filamentous forms.

2.  A method according to claim 1 characterised in that it comprises steps (2) to (4), step (1) being omitted when the sample containing the yeasts, mainly constituted by blastospores, has a concentration higher than or equal to $10^4$ yeasts/ml, preferably a concentration higher than or equal to $10^5$ yeasts/ml, and is substantially free of or depleted in substances which can interfere in the adherence mechanism of said substance (F) to said yeasts.

3.  A method according to claim 1 or claim 2 characterised in that the filamentation of step (2) is carried out for a period of at least 1 hour at 25-40°C to produce filamentous forms selected from the group comprising germ forms and filamentous forms in the case of yeast strains which undergo filamentation or blastospore-spherule forms in the case of yeast strains which undergo pseudofilamentation.

4.  A method according to claim 1 or claim 2 characterised in that the filamentation of step (2) is carried out for 1 hour to 4 hours at 37°C to produce mainly the germ forms which are substantially constituted by germ-tubes or their blastospore-spherule analogs.

5.  A method according to claim 1 or claim 2 characterised in that the contact period of step (3) is an incubation period of at least 30 minutes at 37°C for the filamentous forms with said substance (F) which has been bound to an inert support.

6.  A method according to claim 5 characterised in that said inert support is a latex.

7.  A method according to claim 5 characterised in that said substance (F) is selected from PDF of fibrinogen (FgDP) and fibrin (FnDP).

8.  A method according to claim 5 characterised in that said substance (F) is selected from the global PDF bound to a latex support.

9.  A method according to any one of claims 1, 2 and 5 characterised in that step (4) comprises a reading measure operation selected from the group consisting of:

(a) observation of any possible adherence of said substance (F) to said filamentous yeast forms under a microscope,
(b) observation of any possible agglutination of the complex
    filamentous forms-F-support and
(c) observation of any possible formation of the complex
    filamentous forms-F or
    filamentous forms-F-support using an enzymatic substrate specific for yeast enzymes.

10. A method according to claim 9 characterised in that observation (c) uses an enzymatic substrate which is specific for enzymes having a leucine-arylamidase activity.

11. A method according to claim 10 characterised in that said enzymatic substrate is H-L-Leu-pNA.

12. A method according to claim 9 characterised in that observation (b) uses the agglutination of the complex GT-FDP-latex where GT represents the germ-tubes or their blastospore-spherule analogs.

13. A use of a method according to any one of claims 1 to 12 to determine the affinity of a substance (F) for the filamentous forms of yeast strains in human or veterinary clinical therapy, in agriculture and in the food industry.

14. A use according to claim 13 to determine the pathogenic or non-pathogenic character of yeast strains to be tested.

15. A method for determining the pathogenic or non-pathogenic character of yeast strains to be tested in accordance with the use claimed in claim 14, characterised in that it comprises:

(1) if necessary, multiplication of the blastospores of a sample of yeast strains to be tested to obtain a population of at least $10^4$ yeasts/ml, preferably a population higher than or equal to $10^5$ yeasts/ml,

(2) filamentation of the blastospores,

(3) incubation of the filamentous forms thus obtained with said substance (F) bound to an inert support, preferably global PDF bound to latex, and

(4) observation of the complex which may be formed of the formula by agglutination filamentous forms-F-support.

**16.** A method according to claim 15 characterised in that the blastospore multiplication of step (1) or the blastospore filamentation of step (2) is carried out by hemoculture in a liquid medium, preferably an aerobic medium, from a blood or plasma sample containing test yeast strains previously diluted to a $x^{-1}$ dilution wherein $\underline{x}$ is a number greater than or equal to 50, to eliminate substantially all interference with other substances contained in the plasma which could interfere in the adherence mechanism of said substance (F) to said yeasts, the interfering substances possibly including in particular (i) Fg, Fn, FgDP and FnDP, and (ii) inflammation proteins, antifungal compounds, which may have been administered, and anti*(Candida)* antibodies.

## Patentansprüche

**1.** Verfahren zum Nachweis der möglichen Affinität einer Substanz (F), ausgewählt aus der Gruppe, bestehend aus Fibrinogen (Fg), Fibrin (Fn), ihren Abbauprodukten (FDP) und ihren Gemischen, gegenüber filamentären Formen von Hefestämmen, wobei das Verfahren, welches das Zusammenbringen der filamentären Formen mit der Substanz (F), um diejenigen Hefestämme, die die Substanz (F) binden, von denjenigen zu unterscheiden, die sie nicht binden, umfaßt, dadurch gekennzeichnet ist, daß es die Stufen umfaßt,

bestehend aus

(1.) Kultivieren von (A) einer Probe, die die zu testenden Hefen im wesentlichen in Form von Blastosporen enthält, die wenigstens eine Substanz enthalten können, die in den Bindungsmechanismus der Substanz (F) an die Hefen eingreifen kann und geeignetermaßen so verdünnt ist, daß im wesentlichen das gesamte Eingreifen in den Mechanismus ausgeschaltet ist, in (B) einem Nährmedium, welches die Vermehrung der Blastosporen begünstigt, bis eine Population von größer oder gleich $10^4$ Hefen/ml erhalten wird,

(2.) Kultivieren der so erhaltenen Blastosporen der Hefen in einem Nährmedium, das die Filamentbildung begünstigt,

(3.) Zusammenbringen der so erhaltenen filamentären Formen mit einer Substanz (F), ausgewählt aus der Gruppe, bestehend aus Fibrinogen, Fibrin, FDP und ihren Gemischen, und anschließend

(4.) Bestimmen der Bindung oder der Nichtbindung der Substanz (F) an die filamentären Formen.

**2.** Verfahren nach Anspruch 1, dadurch gekennzeichnet, daß es die Stufen (2.) bis (4.) umfaßt, wobei die Stufe (1.) weggelassen werden kann, wenn eine Probe verfügbar ist, die Hefen enthält, die im wesentlichen aus Blastosporen in einer Konzentration von größer oder gleich $10^4$ Hefen/ml und besser von größer oder gleich $10^5$ Hefen/ml bestehen, und die im wesentlichen einen Mangel an einer Substanz aufweist oder an ihr verarmt ist, die in den Bindungsmechanismus der Substanz (F) an die Hefen eingreifen kann.

**3.** Verfahren nach irgendeinem der Ansprüche 1 und 2, dadurch gekennzeichnet, daß die Filamentbildung in Stufe (2.) während der Dauer von wenigstens 1 Stunde bei 25 bis 40 °C durchgeführt wird, um die filamentären Formen zu erhalten, ausgewählt aus der Gruppe, umfassend die keimfähigen Formen und die filamentären Formen im Falle von Hefestämmen, die Filamente bilden, oder die Blastosporen-Kügelchen-Formen im Falle von Hefestämmen, bei denen eine Pseudo-Filamentbildung stattfindet.

**4.** Verfahren nach irgendeinem der Ansprüche 1 und 2, dadurch gekennzeichnet, daß die Filamentbildung von Stufe (2.) während 1 bis 4 Stunden bei 37 °C durchgeführt wird, um hauptsächlich die keimfähigen Formen zu erhalten, die im wesentlichen aus den keimfähigen Schläuchen oder ihren Analoga, den Blastosporen-Kügelchen, bestehen.

**5.** Verfahren nach irgendeinem der Ansprüche 1 und 2, dadurch gekennzeichent, daß das Zusammenbringen der Stufe (3.) in einer Inkubation während wenigstens 0,5 Stunden bei 37°C der filamentären Formen mit der Substanz (F) besteht, die zuvor an einen inerten Träger gebunden worden ist.

**6.** Verfahren nach Anspruch 5, dadurch gekennzeichnet, daß der inerte Träger ein Latex ist.

**7.** Verfahren nach Anspruch 5, dadurch gekennzeichnet, daß die Substanz (F) ausgewählt wird aus der Gruppe der FDP von Fibrinogen (FgDP) und Fibrin (FnDP).

**8.** Verfahren nach Anspruch 5, dadurch gekennzeichnet, daß die Substanz (F) ausgewählt wird aus der Gruppe der gesamten FDP, die an einen Latexträger gebunden sind.

**9.** Verfahren nach irgendeinem der Ansprüche 1, 2 und 5, dadurch gekennzeichnet, daß die Stufe (4.) einen Abtastvorgang umfaßt, ausgewählt aus der Gruppe, bestehend aus

(a) der mikroskopischen Beobachtung der möglichen Bindung der Substanz (F) an die filamentären Formen der Hefen,
(b) der Beobachtung der möglichen Agglutination des Komplexes
filamentäre Formen-F-Träger und
(c) der Beobachtung der möglichen Bildung des Komplexes
filamentäre Formen-F oder
filamentäre Formen-F-Träger durch ein für Hefeenzyme spezifisches Enzymsubstrat.

**10.** Verfahren nach Anspruch 9, dadurch gekennzeichnet, daß die Beobachtung (c) einfür diejenigen Enzyme spezifisches Enzymsubstrat verwendet, die eine Leucin-Arylamidase-Aktivität aufweisen.

**11.** Verfahren nach Anspruch 10, dadurch gekennzeichnet, daß das Enzymsubstrat H-L-Leu-pNA ist.

**12.** Verfahren nach Anspruch 9, dadurch gekennzeichnet, daß bei der Beobachtung (b) die Agglutination des Komplexes
GT-FDP-Latex verwendet wird, wobei GT die keimfähigen Schläuche oder ihre Analoga, die Blastosporen-Kügelchen, darstellen.

**13.** Anwendung des Verfahrens nach irgendeinem der Ansprüche 1 bis 12 zur Bestimmung der Affinität einer Substanz (F) gegenüber den filamentären Formen von Hefestämmen, im Bereich der klinischen Human- oder Veterinärtherapie, im Bereich der Landwirtschaft und im Lebensmittelbereich.

**14.** Anwendung nach Anspruch 13 zur Bestimmung des pathogenen oder nicht-pathogenen Charakters der zu testenden Hefestämme.

**15.** Verfahren zur Bestimmung des pathogenen oder nicht-pathogenen Charakters der zu testenden Hefestämme entsprechend der Anwendung nach Anspruch 14, dadurch gekennzeichnet, daß es umfaßt

(1.) falls notwendig, die Vermehrung der Blastosporen einer Probe der zu testenden Hefestämme bis eine Population von wenigstens $10^4$ Hefen/ml und besser eine Population von größer oder gleich $10^5$ Hefen/ml erhalten wird,
(2.) die Filamentbildung der Blastosporen,
(3.) die Inkubation der so erhaltenen filamentären Formen mit der Substanz (F), die an einen inerten Träger gebunden ist, vorzugsweise mit den an Latex gebundenen Gesamt-FDP, und
(4.) die Beobachtung des gegebenenfalls gebildeten Komplexes der Formel
filamentäre Formen-F-Träger durch Agglutination.

**16.** Verfahren nach Anspruch 15, dadurch gekennzeichnet, daß die Vermehrung der Blastosporen in Stufe (1.) oder die Filamentbildung der Blastosporen in Stufe (2.) durch Hämokultur in einem flüssigen Medium, vorzugsweise aerob, durchgeführt wird, ausgehend von einer Blut- oder Plasmaprobe, die die zu testenden Hefestämme enthält, und die zuvor gemäß einer Verdünnung von $x^{-1}$ verdünnt worden ist, wobei x eine Zahl von größer oder gleich 50 bedeutet, um im wesentlichen die gesamte Störung durch die anderen Substanzen auszuschalten, die in dem Plasma enthalten sind und in den Bindungsmechanismus der Substanz (F) an die Hefen eingreifen können, wobei dieinterferierenden Substanzen insbesondere Fg, Fn, FgDP und FnDP einerseits und die Entzündungsproteine, die fungiziden Verbindungen, die verabreicht worden sein könnten, die Anti(candida)-Antikörper andererseits umfassen können.